# EUROPEAN PATENT SPECIFICATION

(11) **EP 3 372 235 B1**
(45) Date of publication and mention of the grant of the patent: **30.09.2020**
(21) Application number: 16862232.2
(22) Date of filing: 07.11.2016
(51) Int. Cl.: A61K 35/644, A61K 31/015, A61P 25/28, A61P 43/00

(54) **ENHANCER OF ANTI-DEMENTIA ACTIVITY OF ROYAL JELLY**
VERSTÄRKER DER AKTIVITÄT GEGEN DEMENZ VON GELEE ROYALE
AGENT D'AUGMENTATION DE L'ACTIVITÉ ANTI-DÉMENCE DE LA GELÉE ROYALE

(30) Priority: 05.11.2015 JP 2015217639
(43) Date of publication of application: 12.09.2018
(73) Proprietor: Japan Royal Jelly Co., Ltd., Tokyo 163-0636 (JP); Tohoku University, Sendai-shi, Miyagi 980-8577 (JP)
(72) Inventor: OHIZUMI, Yasushi, Sendai-shi Miyagi 989-3201 (JP); TAKITO, Jiro, Tokyo 142-8555 (JP); YAMAGUCHI, Kikuji, Tokyo 163-0636 (JP); INOMATA, Hiroshi, Sendai-shi Miyagi 980-8577 (JP); OTA, Masaki, Sendai-shi Miyagi 980-8577 (JP)
(74) Representative: Hoffmann Eitle
(86) International application number: PCT/JP2016/082932
(87) International publication number: WO 2017/078175

(56) References cited:
- EP-A1- 2 311 472
- WO-A1-2009/154197
- JP-A- 2007 302 572
- HIRONORI FUJIWARA ET AL: "Honeybee Royal Jelly and Nobiletin Stimulate CRE-Mediated Transcription in ERK-Independent and -Dependent Fashions, Respectively, in PC12D Cells", JOURNAL OF PHARMACOLOGICAL SCIENCES, vol. 116, no. 4, 1 January 2011 (2011-01-01), pages 384-387, XP055587364, JP ISSN: 1347-8613, DOI: 10.1254/jphs.11001SC

## Description

### TECHNICAL FIELD

The present invention is related to enhancers of anti-dementia activity of royal jelly including limonene, as set forth in the claims.

### BACKGROUND ART

In Japan which is turning into a super-aging society, prevention and treatment of cognitive impairments caused by aging such as problems with concentration, memory, organization, planning, and problem solving, in particular, Alzheimer's disease which is a representative disease of the same, is a significant social issue.

Limonene is a monocyclic monoterpenoid contained in a large amount in many citrus peels. This is one of the substances forming a part of the composition of the fragrance of citrus, which is used as perfume, and it is also commercially available in chemical synthetic products.

Royal jelly is a secretion from the pharyngeal glands of young worker honeybees, which is fed as a food to queen-to-be larvae and a grown-up queen bee. This is the only energy source throughout the entire life of the queen bee who lives 40 times longer than the worker bees. Moreover, it is known that royal jelly contains a large amount of vitamins, minerals, and amino acids, which are incomparable to those of honey and it is rich in proteins and contains various nutrients. In particular, as royal jelly indicates an extremely superior effect to learning disability models, it is known to be useful as an improving agent of cognitive impairment such as Alzheimer's disease (Patent Document 1).

Patent Document 2 concerns a cognitive impairment ameliorant being useful for the prevention or treatment of cognitive impairment, and particularly Alzheimer's disease. The cognitive impairment ameliorant contains royal jelly as an active ingredient thereof.

Patent Document 3 relates to a brain function improver containing at least 1 kind selected from citral, linalool, nootkatone, limonene, perillyl alcohol and perilla acid as an active ingredient being excellent in a nerve growth factor-reinforcing activity and memory learning function-improving activity, and a brain function-improving composition containing the same.

Non-patent Document 1 describes honeybee royal jelly and Nobiletin stimulate CRE-mediated transcription in ERK-independent and -dependent fashions, respectively, in PC12D cells.

### PRIOR ART DOCUMENTS

### PATENT DOCUMENTS

Patent Document 1: WO2009/154197 A
Patent Document 2: EP 2 311 472 A1
Patent Document 3 JP 2007 302572 A
Non-patent Document 1: HIRONORI FUJIWARA ET AL, JOURNAL OF PHARMACOLOGICAL SCIENCES, vol. 116, no. 4, 1 January 2011 (2011-01-01), pages 384-387

### SUMMARY OF INVENTION

### Technical Problem

The problem of the present invention is to provide an enhancer of anti-dementia activity of royal jelly.

### SOLUTION TO PROBLEM

The present inventors have carried out dedicated research and as a result of this, and they have found out that limonene remarkably enhances an anti-dementia effect shown by royal jelly, and thus, completed the present invention.

### EFFECTS OF THE INVENTION

The present invention is related to the following:
[1] Limonene for use in a method of improving a cognitive disorder, wherein the method includes oral or parenteral administration of an agent comprising limonene and royal jelly ;
[2] an agent comprising limonene and royal jelly for use in improving a cognitive disorder, wherein limonene is used to increase the anti-dementia activity of royal jelly.

### BRIEF DESCRIPTION OF THE DRAWINGS

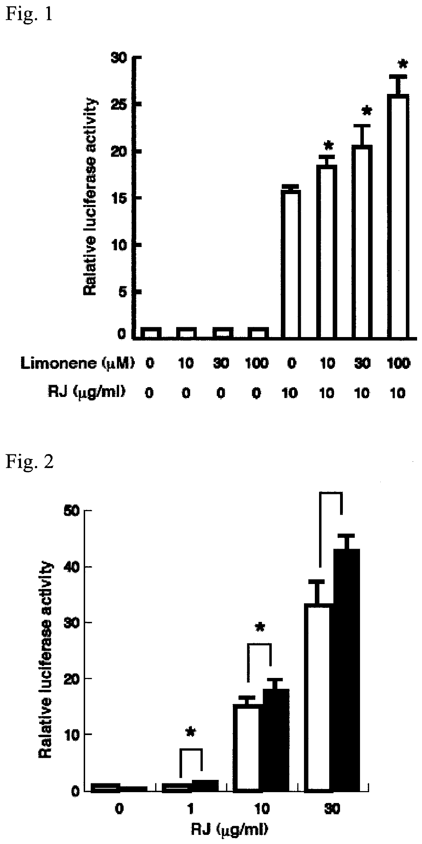

Fig. 1: Figure 1 shows the effect of limonene and limonene and royal jelly in combination on CRE-dependent transcription activity in PC12 cells. Data are expressed as mean ± standard deviation (n = 4). * shows having a significant difference to the luciferase activity of royal jelly alone (p <0.05). The vertical axis shows the relative luciferase activity. RJ indicates royal jelly.
FIG. 2: Figure 2 shows the effect of 100 µM limonene on CRE dependent transcription activity of royal jelly at various concentrations in PC12 cells. Data are expressed as mean ± standard deviation (n = 4). * shows that the combined use of limonene demonstrates a significant difference as compared to the luciferase activity of royal jelly used alone (p <0.05). The vertical axis shows the relative luciferase activity. RJ indicates royal jelly. □ shows the activity of royal jelly alone. ■ shows the activity at the time of adding 100 µM limonene to the royal jelly.

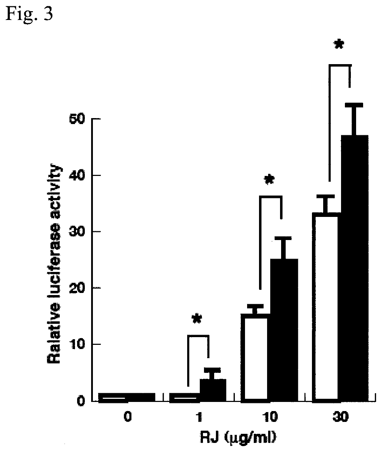

FIG. 3: Figure 3 shows the effect of 200 µM limonene on CRE dependent transcription activity of royal jelly at various concentrations in PC12 cells. Data are expressed as mean ± standard deviation (n = 4). * shows that the combined use of limonene demonstrates a significant difference as compared to the luciferase activity of royal jelly when used alone (p <0.05). The vertical axis shows the relative luciferase activity. RJ indicates royal jelly. □ shows the activity of royal jelly alone. ■ shows the activity at the time of adding 100 µM limonene to the royal jelly.

### DESCRIPTION OF EMBODIMENTS

Limonene of the present invention may be chemically synthesized by using well-known methods to those skilled in the art. In addition, it can also be extracted from the citrus family plant. Any of D form, L form, or racemate of limonene can be used in the present invention.

The citrus family plant may include such as Hirami lemon (Citrus depressa), Satsuma mandarin (Citrus unshiu), Oobenimikan (Citrus tangerina), Kobenimikan (Citrus erythrosa), Daidai (Citrus aurantium), Mediterranean mandarin (Citrus deliciosa), Zabon (Citrus grandis), Hirakishu (Citrus kinokuni), Ponkan (Citrus reticulata), Tachibana (Citrus tachibana), and Sankitsu (Citrus sunki).

Extracts of the citrus family plant used in the present invention may be extracted from either the fresh state after harvesting or the dried state. Parts may include such as mature or immature fruits, fruit peels, seeds, leaves, leaf stalks, branches, roots, and flowers. Preferably, they are from fruits and fruit peels.

Royal jellies that can be used in the present invention are not particularly limited, and commercially available royal jellies can be used. Preferably, products from JAPAN ROYAL JELLY Co., Ltd can be provided.

In the present invention, the content of limonene is 0.0002 wt% to 0.10 wt%, preferably from 0.0005 wt% to 0.05 wt%, more preferably from 0.001 wt% to 0.01 wt%. On the other hand, when using an extract containing 0.5% of limonene, it is 0.004 wt% to 2.0 wt%, preferably from 0.01 wt% to 1.0 wt%, more preferably 0.02 wt% to 0.2 wt%. Moreover, as a royal jelly FD powder, the content of royal jelly is 0.001 wt% to 60 wt%, preferably 0.01 wt% to 45 wt%, and more preferably from 0.1 wt% to 30 wt%.

In the present invention, the ratio of limonene content and royal jelly content is 1 : 5 to 1 : 300,000, preferably 1 : 20 to 1 : 9000, more preferably 1 : 100 to 1 : 3000. On the other hand, when using an extract containing 0.5% of limonene, it is 4 : 1 to 1 : 15000, preferably 1 : 1 to 1 : 450, more preferably 1 : 5 to 1 : 150.

The cognitive impairment improving agent of the present description may be formulated in pharmaceuticals, quasi drugs, food, or the like. Moreover, the present invention could be used for either oral administration or parenteral administration.

The dosage of the cognitive impairment improving agent of the present description is not particularly limited; however, for example, in case of oral administration, it is 10 - 60 mg/kg of body weight per day, preferably 20 mg/kg of body weight per day, and in case of parenteral administration, it is 2 - 6 mg/kg of body weight per day, preferably 2 - 3 mg/kg of body weight per day. The above-mentioned dosage can be administered in a single dose or divided into 2 - 3 times a day, and depending on the age, disease state, and symptoms, it may be appropriately increased or decreased.

Specific examples of the additives include such as maltitol, lactose, dextrin, sucrose, mannitol, corn starch, sorbitol, crystalline cellulose, and polyvinylpyrrolidone, and these may be used alone or in combination as appropriate. According to the description of the Japanese Pharmacopoeia, these pharmaceuticals can be prepared by a method which is suitable for the form of each pharmaceutical. Moreover, flavoring agents, coloring agents, sweetening agents, and the like, may be appropriately used. The content of these additives can be appropriately chosen by those skilled in the art.

Forms of quasi drugs include such as tablets, capsules, granules, jellies, and drinks. These quasi drugs can be prepared using additives of the conventional quasi-drug preparation. Moreover, these quasi drugs, for example, can also contain other active ingredients such as vitamins. Furthermore, additives such as sweetening agents, flavoring agents, coloring agents, and antioxidants may be used alone or in combination as appropriate. These quasi drugs can be prepared by methods well-known to those skilled in the art.

The forms of food include such as noodles, pastas, granules, tablet candies, jellies and liquids (beverages). These foods can be prepared by appropriately using a variety of food materials. Specific examples of food ingredients include such as rice, wheat, corn, potato, sweet potato, soybean flour, seaweed powder, syrup, lactose, glucose, fructose, sucrose, and mannitol, and these may be used alone or in combination as appropriate. If necessary, it can have a desired shape by using such as water, if necessary. Furthermore, flavoring agents, coloring agents, sweetening agents, cooking oils, and vitamins may be added as appropriate.

Hereinbelow, the present invention is specifically explained in the Examples; however, this is merely an illustration of the representative examples, and the present invention is not limited to these examples.

### EXAMPLE 1

A cAMP/PKA/ERK/CREB dependent signaling pathway enhances the CRE-dependent transcription activity and allows the expression of a specific gene, and it is demonstrated to form a memory/learning ability of the brain. In PC12 cells which are believed to be model cells of neuronal cells, it is known that a royal jelly which is a cognitive impairment improving agent elevates the CRE-dependent transcription activity via cAMP/PKA/ERK/CREB pathway (Patent Document 1). In other words, it is believed that a substance which elevates the CRE-dependent transcription activity in PC12 cells is useful as a cognitive impairment or Alzheimer's disease improving agent.

An effect of limonene on CRE-dependent transcription activity by royal jelly in PC12 cells is examined by a CRE reporter gene assay.

### 1. Experimental Methods

### Culturing PC12 cells

PC12 cells (RIKEN BRC) was inactivated (56 °C, 30 minutes) and 10% fetal bovine serum, 10% horse serum, Dulbecco's modified Eagle medium (Advanced DMEM: Gibco) supplemented with penicillin (50 units/ml) and streptomycin (50 µg/ml) was used for subculturing. The cells were cultured in an incubator containing 95% Air - 5% CO₂ heated at 37 °C.

### The preparation of royal jelly aqueous solution

Raw royal jelly was collected from Yamaguchi Kikuchi's natural beekeeping method (JAPAN ROYAL JELLY Co., Ltd). The freeze-dried powder of raw royal jelly was dissolved in PBS and made into a 25% (w/v) solution, and this was stirred overnight at 4 °C. Then, this was centrifuged for 10 minutes at 12,000 × g, the supernatant was filter sterilized, and stored frozen at - 20 °C after dispensing. The protein concentration in the royal jelly was quantified using the BCA protein assay (Pierce).

### The D-limonene used in the present experiment was purchased from Nacalai tesque, INC.

### CRE reporter gene assay

Hundred µl of 10% fetal calf serum-containing culture solution containing 10,000 of PC12 cells was seeded in a 96-well plate and cultured for 24 hours. Per well, 0.1 µg pCRE (luciferase reporter plasmid with CRE sequences) and 0.02 µg phRG-TK (renilla luciferase plasmid) were transiently introduced to cells using LipofectAMINE 2000, after 6 hours, the culture solution was replaced with Dulbecco's modified Eagle's medium (Advanced DMEM: Gibco) supplemented with 1% fetal bovine serum, 1% horse serum, penicillin (50 units/ml) and streptomycin (50 µg/ml). Eighteen hours later, royal jelly or limonene was added and this was stimulated for 5 hours. After collecting the cell solution, Dual luciferase kit (Promega) was used and luciferase activity was measured with a luminometer. Luciferase activity was corrected with renilla luciferase activity to normalize the plasmid introduction efficiency.

### Results

### The effect of royal jelly and limonene on CRE-dependent transcription activity in PC12 cells

PC 12 cells were seeded on a 96-well plate at a cell density of 1 × 10⁴ cells/well and cultured overnight. CRE-dependent reporter plasmid was introduced and the cells were cultured for 24 hours followed by a treatment with royal jelly and limonene for 5 hours. The treated cells were collected and the CRE-dependent transcription activity in cell extract solution was measured as luciferase activity.

As a result of this, in PC12 cells, limonene was found to promote the CRE-dependent transcription activity demonstrated by the royal jelly in a concentration dependent manner. Here, a matter of importance is that, even though limonene alone used in the concentration range of this experiment does not at all have the CRE-dependent transcription activity, it enhanced the CRE-dependent transcription activity when used in combination with royal jelly (Fig. 1). CRE-dependent transcription reaction in neurons is a key mechanism of long-term potentiation of synaptic signaling, which is one of the fundamental cellular mechanisms of memory and learning in the brain, and the elevation of CRE-dependent transcription activity is believed to promote memory formation. That is to say that limonene enhanced anti-dementia activity of royal jelly.

Then, the effect of limonene in royal jelly of various concentrations was examined. 100 µM of limonene equally enhanced the luciferase activity of 1, 10, 30 µg/ml of royal jelly (Fig. 2). In particular, 1 µg/ml of royal jelly alone did not demonstrate a significant elevation in luciferase activity; however, the activity was elevated with a significant difference by using limonene in combination. This suggests that a low concentration royal jelly has a significant anti-dementia activity when combinedly used with limonene. In experiments with 200 µM of limonene, a more prominent effect of limonene was obtained (Fig. 3).

### INDUSTRIAL APPLICABILITY

Enhancers of anti-dementia activity of royal jelly including limonene of the present invention are useful for the improvement of cognitive impairments.

## Claims

1. Limonene for use in a method of improving a cognitive disorder, wherein the method includes oral or parenteral administration of an agent comprising limonene and royal jelly.

2. An agent comprising limonene and royal jelly for use in improving a cognitive disorder wherein limonene is used to increase the anti-dementia activity of royal jelly.

## Patentansprüche

1. Limonen zur Verwendung in einem Verfahren zur Besserung einer kognitiven Störung, wobei das Verfahren die orale oder parenterale Verabreichung eines Mittels, umfassend Limonen und Gelee royale, einschließt.

2. Mittel, umfassend Limonen und Gelee royale, zur Verwendung für die Besserung einer kognitiven Störung, wobei Limonen verwendet wird, um die Aktivität von Gelee royale gegen Demenz zu erhöhen.

## Revendications

1. Limonène destiné à être utilisé dans un procédé pour améliorer un trouble cognitif, dans lequel le procédé inclut une administration par voie orale ou par voie parentérale d'un agent comprenant du limonène et de la gelée royale.

2. Agent comprenant du limonène et de la gelée royale destiné à être utilisé pour améliorer un trouble cognitif dans lequel le limonène est utilisé pour augmenter l'activité anti-démence de la gelée royale.
